(19) Europäisches Patentamt
European Patent Office
Office européen des brevets

(11) **EP 4 786 448 A1**

(12) **EUROPEAN PATENT APPLICATION**

(43) Date of publication:
**05.08.2026 Bulletin 2026/32**

(21) Application number: **25275007.0**

(22) Date of filing: **03.02.2025**

(51) International Patent Classification (IPC):
**C07C 201/08** (2006.01)   **C07C 205/06** (2006.01)

(52) Cooperative Patent Classification (CPC):
(C-Sets available)
**C07C 201/08**   (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **BAE SYSTEMS plc
London SW1Y 5AD (GB)**

(72) Inventor: **The designation of the inventor has not
yet been filed**

(74) Representative: **BAE SYSTEMS plc
Group IP Department
Victory Point
Frimley
Camberley, Surrey GU16 7EX (GB)**

(54) **FLOW SYNTHESIS METHOD**

(57)    The present invention relates to methods of producing energetic materials by flow synthesis. There is provided a method for the synthesis of 2,4,6-trinitrotoluene (TNT) using a flow reactor, the method comprising the steps of:

a) providing an input flow of sulphuric acid comprising at least 50 wt% sulphuric acid;

b) providing an input flow of nitric acid comprising at least 50 wt% nitric acid;

c) providing an input flow of toluene;

d) directing the input flow of sulphuric acid, the input flow of nitric acid and the input flow of toluene through a first flow path in a first zone of the flow reactor, the first zone having a temperature $T^1$, to provide a first output flow;

e) directing the first output flow through a second flow path in a second zone of the flow reactor, the second zone having a temperature $T^2$, to provide a second output flow comprising TNT;

wherein the temperature $T^2$ is greater than the temperature $T^1$.

Fig. 1

**(Cont. next page)**

(52) Cooperative Patent Classification (CPC): (Cont.)

C-Sets
**C07C 201/08, C07C 205/06**

**Description**

FIELD

The present invention relates to methods of producing energetic materials by flow synthesis. In particular, the present invention relates to methods of nitrating toluene to produce 2,4,6-trinitrotoluene (TNT), using a flow reactor.

BACKGROUND

2,4,6-trinitrotoluene, commonly known simply as trinitrotoluene or TNT, is produced globally in large volumes for military and industrial uses. It is estimated that the global production of TNT will reach over 30 kilotons in 2025. TNT is produced on a large scale in batch processes involving three separate steps of sequential nitration of toluene, mononitrotoluene (MNT) and dinitrotoluene (DNT), using sulphuric acid and nitric acid. The TNT so produced is then purified by crystallisation and / or stabilised by removing the by-product isomers of TNT, which are less stable and lead to handling issues.

This batch manufacturing process inevitably involves the production and keeping of large quantities of the intermediates MNT and DNT and the potentially unstable final TNT material, in a manufacturing site. Due to the inherent potential of TNT to energetically decompose, such large concentrations of said materials present safety risks to personnel, plant and surrounding infrastructure, which can be difficult and costly to mitigate. Also, current methods for preparing TNT produce a large amount of liquid waste known as redwater, generated from a sulfiting step used to remove unwanted TNT isomers. This redwater waste is harmful to the environment and therefore presents difficulties and incurs significant costs for disposal.

Therefore it would be advantageous if highly energetic materials such as TNT could be produced in a way which significantly reduces the risks associated with large volumes of such materials accumulating at a manufacturing site and / or requiring bulk transportation to a site of subsequent use, for example for incorporation into ordnance.

It may also be advantageous to manufacture such highly energetic materials in a way which does not require the isolation and storage of large quantities of intermediate materials.

It would also be advantageous to eliminate the production of significant amounts of waste products, in particular redwater.

SUMMARY

Before the present invention is described in further detail, it is to be understood that the invention is not limited to the particular embodiments described, and as such may, of course, vary. It is also to be understood that the terminology used herein is for the purpose of describing particular embodiments only, and is not intended to be limiting, since the scope of the present invention will be limited only by the appended claims.

According to a first aspect of the present invention, there is provided a method for the synthesis of 2,4,6-trinitrotoluene (TNT) using a flow reactor, the method comprising the steps of:

a) providing an input flow of sulphuric acid comprising at least 50 wt% sulphuric acid;

b) providing an input flow of nitric acid comprising at least 50 wt% nitric acid;

c) providing an input flow of toluene;

d) directing the input flow of sulphuric acid, the input flow of nitric acid and the input flow of toluene through a first flow path in a first zone of the flow reactor, the first zone having a temperature $T^1$, to provide a first output flow;

e) directing the first output flow through a second flow path in a second zone of the flow reactor, the second zone having a temperature $T^2$, to provide a second output flow comprising TNT;

wherein the temperature $T^2$ is greater than the temperature $T^1$.

The inventors have found that the method of this first aspect can efficiently produce TNT in a purity suitable for subsequent use. The TNT is produced in a continuous (flow) process, without isolation of intermediate materials such as MNT and DNT and therefore avoiding the inherent dangers of forming large amounts of energetic materials in a single reaction vessel, in a relatively high yield. Also, the method may advantageously avoid the use of hundreds of litres of highly concentrated acid in a large reactor vessel in a batch process, which would also present inherent safety risks.

This method may therefore enable the continuous production of TNT in relatively small amounts compared to known batch-wise methods and therefore reduce or avoid the accumulation of large amounts of TNT at a manufacturing site, which is the inevitable result of such known batch processes. The TNT so produced may be continuously removed from the process equipment and safely stored elsewhere or incorporated into products and then safely stored.

The method of this first aspect therefore has the potential to improve the safety profile of TNT manufacture and supply. The method may also provide an annual output of TNT equivalent to that produced by established processes and sites, using a site and plant with a significantly smaller footprint and potentially a lower overall cost. Alternatively, current manufacturing sites may be able to operate the method of the present invention to produce a larger annual output of TNT without increasing safety risks. For example, such sites operating the method of the present invention to produce TNT may be able to operate with significantly reduced safety exclusion zones around the manufacturing site, which may be advantageous for the practicalities and costs of operating such sites, and may lead to cost advantages and efficiencies in the use / incorporation of the TNT into other products, downstream of the manufacturing process.

In particular, the inventors have found that the method of this first aspect wherein the temperature $T^2$ of the second zone of the flow reactor is greater than the temperature $T^1$ of the first zone of the flow reactor, can provide an improved yield and purity of TNT compared to similar methods wherein the reaction from toluene to TNT is carried out at a single set temperature in a flow reactor (and therefore in a single reaction zone of a flow reactor). Such methods may provide TNT products containing significant amounts of unreacted DNT and / or unwanted isomeric by-products, which adversely affects the quality of the TNT, in its intended further uses. The use of said first and second zones having the temperatures $T^1$ and $T^2$ advantageously allows a first reaction, for example the nitration of toluene to DNT, to be carried out at a favourable temperature for said reaction and subsequent reactions, for example the further nitration of DNT to TNT to be carried out at a different, higher temperature which is more favourable for said reactions. The method of this first aspect allows said reactions to be carried out at different temperatures without isolating the intermediate products and subjecting said intermediate products to a second, separate reaction. In particular, the method of this first aspect allows a relatively high temperature to be used in the second zone which is particularly suitable for the further nitration of DNT to TNT, when said temperature would be too high for an efficient nitration of toluene to DNT.

Furthermore, the method of this first aspect advantageously reduces the production of significant amounts of waste products, in particular redwater.

The method of this first aspect is carried out using a flow reactor. A suitable flow reactor is configured to accommodate at least three input flows, to provide the input flows specified in steps a), b) and c). The flow reactor suitably comprises at least two zones comprising flow paths for the reagents within the flow reactor; for example at least a first flow path in a first zone and a second flow path in a second zone, wherein the temperatures in the first and second zones are independently controllable to achieve the different temperature $T^1$ and $T^2$ of steps d) and e).

Suitably the flow reactor comprises a mixing zone, comprising at least one mixing plate to mix together the input flows of steps a), b) and c). The mixing zone is where the input flow reagents are brought together to be mixed in order to start a chemical reaction. The flow reactor may comprise more than one mixing zone. Suitably the mixing zone is part of the first zone. Suitably the first zone of the flow reactor is a mixing zone, preferably comprising a mixing plate.

The mixing of chemicals, and typically the case for the synthesis of energetic materials, is an exothermic reaction. Whilst the mixing plate will experience an exothermic reaction, the initial transfer into a residence plate (i.e. the core of the flow reactor where the reaction takes place), the initial reaction may still be highly exothermic and the removal of excess heat, may help to control the reaction, especially from a safety perspective.

The mixing plates and/or residence plates of the flow reactor may be selected from any high thermally conductive material, such as metal or conductive ceramic. The method of this first aspect involves the use of highly corrosive acids, nitric and or sulphuric acids, so the mixing plates and/or residence plates are preferably inert with respect to the reagents, i.e. resistant to corrosion by means of contact with the reagents and impurities or combinations thereof. Preferably a suitable engineering conductive ceramic is silicon carbide.

In the method of this first aspect, mixing of the input flows of steps a), b) and c) suitably initiates at least a first reaction of the toluene with the acids. Therefore at least said first reaction suitably takes place in the mixing zone of the first zone of the flow reactor, in addition to the mixing of said input flows.

The first zone may comprise a residence plate, in addition to the mixing plate. Said residence plate is suitably arranged after the mixing plate (i.e. downstream in the flow reactor) to provide additional volume of the first flow path for the mixed input flows, in which the first reaction can take place. In such embodiments, said residence plate suitably has the temperature $T^1$.

The flow reactor suitably comprises a reaction zone. Said reaction zone suitably comprises at least one residence plate which forms a microchannel path in which the reaction occurs.

A reaction zone is where the reaction proceeds to take place with the mixed input flow reagents, from the mixing zone. The reaction zone may have any number of residence plates, the number of plates determines the physical length of the micro flow channel, and therefore the reaction time. The transition time for the mixed input flow reagents through the reaction zone, may be determined by the length of micro flow channel, i.e. the number of residence plates, and also by the pressure (especially where gas evolution is involved) - i.e. the flow rate. The temperature of the second (reaction) zone is elevated compared to the first zone, to drive the reaction to completion and obtain higher yields, and quicker throughput times.

Suitably the second zone of the flow reactor is a reaction zone, suitably comprising at least one residence plate. The second zone may comprise more than one residence plate, for example 2, 3 or 4 residence plates.

In some embodiments, the second zone may comprise a mixing plate (i.e. a second mixing plate of the flow reactor). Said mixing plate may allow a further input flow, for example a further input flow of nitric acid, to be mixed with the first output flow from the first zone.

The temperatures of the first and second zones of the flow reactor, and any further zones, may be held at a selected temperature by any suitable heating or cooling means, such as by electrical heaters, water/oil circulators, heat exchangers, clearly naked flames are possible, but are not safe practice.

Preferably the first zone comprises a first water circulator and the second zone comprises a second water circulator. Preferably each of the first and second zones comprises its own heating or cooling means. The temperature within the first zone and/or second zone may be actively monitored such that the rate of heating and cooling may be selected to maintain the temperature range within each first, second or further zones of the flow reactor.

Step (a) of the method of this first aspect involves providing an input flow of sulphuric acid. The input flow of sulphuric acid comprises at least 50 wt% sulphuric acid, at least 60 wt%, at least 70 wt% or at least 80 wt% sulphuric acid. The input flow of sulphuric acid suitably comprises at least 90% sulphuric acid (by mass). The input flow of sulphuric acid may comprise at least 95% sulphuric acid, at least 96%, at least 98% or approximately 98% sulphuric acid.

Embodiments wherein the input flow of sulphuric acid comprises at least 90 wt% sulphuric acid are believed to be particularly advantageous, for example in providing TNT with the desired purity and favourable yield.

Step (b) of the method of this first aspect involves providing an input flow of nitric acid. The input flow of sulphuric acid comprises at least 50 wt% nitric acid, at least 60 wt%, at least 70 wt% or at least 80 wt% nitric acid. The input flow of nitric acid suitably comprises at least 90% nitric acid (by mass). The input flow of nitric acid may comprise at least 95% nitric acid, at least 96%, at least 98% or approximately 99% nitric acid.

Embodiments wherein the input flow of nitric acid comprises at least 90 wt% nitric acid are believed to be particularly advantageous, for example in providing TNT with the desired purity and favourable yield.

Suitably the nitric acid is prepared less than one day prior to use in the method, suitably less than 12 hours or less than 6 hours prior to use in the method. Using relatively freshly prepared nitric acid may have a beneficial effect on the yield of TNT produced by the method.

Step (c) of the method of this first aspect involves providing an input flow of toluene. Suitably the input flow of toluene is neat toluene. Suitably said input flow consists essentially of toluene. The toluene suitably has a purity of at least 90%, suitably at least 95%, suitably at least 99%, suitably at least 99.9%.

The flow rates of the input flow of sulphuric acid, the input flow of nitric acid and the input flow of toluene may be independently selected from any suitable flow rate which provides a total nitric acid concentration capable of carrying out the nitration of toluene to produce TNT. The actual flow rate of the input flows of sulphuric acid and nitric acid may be $\mu L$ through to millilitres to litres per minute, depending on the capacity / size of the flow reactor. The flow rates of the input flow of sulphuric acid, the input flow of nitric acid and the input flow of toluene may be selected to achieve a desired molar ratio (equivalents) of the input reagents and/or to achieve a desired overall reaction flow rate through the flow reactor.

The input flow of nitric acid and the input flow of toluene suitably provide at least 3 (molar) equivalents, at least 4 equivalents, at least 5 equivalents or at least 5.5 equivalents of nitric acid, compared to toluene. The input flow of nitric acid and the input flow of toluene suitably provide up to 10, 8 or 7 equivalents of nitric acid, compared to toluene. The input flows suitable provide from 3 to 10 equivalents of nitric acid compared to toluene, suitably from 5 to 7 equivalents. The input flows suitably provide approximately 6 equivalents of nitric acid compared to toluene.

The input flow of sulphuric acid and the input flow of toluene suitably provide at least 3 equivalents, at least 5 equivalents, at least 10 equivalents, at least 20 equivalents or at least 25 equivalents of sulphuric acid, compared to toluene. The input flow of sulphuric acid and the input flow of toluene suitably provide up to 40 or 35 equivalents of sulphuric acid, compared to toluene. The input flows suitably provide from 3 to 40 equivalents of sulphuric acid to toluene, suitably from 20 to 35 equivalents. The input flows suitably provide approximately 33 equivalents of sulphuric acid, compared to toluene.

The input flows suitably provide from 3 to 10 equivalents of nitric acid and 3 to 40 equivalents of sulphuric acid, compared to toluene. In some embodiments, the input flows suitably provide from 5 to 7 equivalents of nitric acid and 30 to 35 equivalents of sulphuric acid, compared to toluene.

In some embodiments, the input flows suitably provide approximately 6 equivalents of nitric acid and approximately 33 equivalents of sulphuric acid, compared to toluene.

The equivalents of the nitric acid, sulphuric acid and toluene used in the method, as discussed above, may be achieved by configuring the flow reactor accordingly, for example by adjusting the flow rates of the input flows.

Step d) of the method of this first aspect involves directing the input flow of sulphuric acid, the input flow of nitric acid and the input flow of toluene through a first flow path in a first zone of the flow reactor having a temperature $T^1$, to

provide a first output flow.

Suitably step d) involves mixing the input flow of sulphuric acid, the input flow of nitric acid and the input flow of toluene. Suitably the input flows of sulphuric acid, nitric acid and toluene are mixed to provide a first reaction flow. Suitably said input flows are mixed in a mixing zone of the flow reactor, the mixing zone suitably comprising at least one mixing plate, as discussed above. Suitably the mixing zone is comprised in the first zone of the flow reactor. Suitably the first zone of the flow reactor is a mixing zone. Suitably the flow reactor is configured to provide the first reaction flow comprising the sulphuric acid, nitric acid and toluene in the first flow path in the first zone of the flow reactor.

Suitably step d) involves combining the input flow of sulphuric acid and the input flow of nitric acid to provide a combined acid flow, and involves combining the combined acid flow with the input flow of toluene. Said sequential mixing provides the first reaction flow.

Suitably said sequential combining of the input flows takes place in the first zone, which is suitably a mixing zone. Therefore the flow reactor is suitably configured to carry out this sequential mixing of the sulphuric acid and nitric acid followed by mixing of the resultant combined acid flow with the input flow of toluene.

Therefore step d) suitably involves the following sub-steps d1) to d3) of:

d1) combining the input flow of sulphuric acid and the input flow of nitric acid to provide a combined acid flow;

d2) combining the combined acid flow with the input flow of toluene to provide a first reaction flow;

d3) directing the first reaction flow through a first flow path in a first zone of the flow reactor, the first zone having a temperature $T^1$, to provide a first output flow.

Suitably steps d1) and d2) are carried out in the first zone of the flow reactor. Suitably steps d1), d2) and d3) are carried out in the first zone of the flow reactor at temperature $T^1$.

Suitably step d) involves directing the first reaction flow through the first flow path in the first zone of the flow reactor having a temperature $T^1$. The first zone of the flow reactor is suitably a mixing zone, as described above. Once the three input flows have been mixed to provide the first reaction flow, a first reaction is initiated. Therefore at least said first reaction takes place in the first zone. Therefore the first zone may be considered a mixing and first reaction zone.

The first flow path of the first zone may be provided by a mixing plate of the flow reactor. Said mixing plate may provide a sufficient volume for the first reaction flow to pass through and undergo at least the first reaction, at temperature $T^1$. The first zone may comprise at least one residence plate, which may be in addition to the mixing plate, in order to increase the length / volume of the first flow path. The first zone may comprise at least one further residence plate, such that the residence plate and the further residence plate are maintained at the temperature $T^1$. The number and sizes of said residence plates may be adjusted according to the desired volume and/or residence time of the first reaction flow in the first flow path.

In the first flow path of the first zone of the flow reactor, at least a first reaction suitably takes place. In said first reaction, the toluene comprised in the first reaction flow is suitably nitrated to produce dinitrotoluene (DNT). Suitably the first output flow comprises DNT. The first output flow suitably comprises at least 90 mol% DNT, at least 95 mol% or at least 97 mol% DNT, with respect to all toluene-derived species present in the first output flow.

The temperature $T^1$ is suitably a temperature sufficient to promote and the reaction of toluene in the first reaction flow with the nitric acid and sulphuric acid to produce DNT. The temperature $T^1$ may be at least 11°C at least 20°C, at least 30°C, at least 50°C, at least 60°C or at least 70°C. Suitably the temperature $T^1$ is below a temperature which promotes the reaction of DNT with the nitric acid and sulphuric acid to form TNT. Suitably the temperature $T^1$ is below the boiling point of toluene, which may inhibit the first and subsequent reactions of the toluene with the combined acid flow. The temperature $T^1$ may be up to 100°C, up to 90°C or up to 85°C.

The temperature $T^1$ is suitably from 20 to 100°C from 30 to 90°C. The temperature $T^1$ may be from 60 to 90°C, from 70 to 85°C or from 75 to 85°C. For example, $T^1$ may be approximately 80°C.

Step e) of the method of this first aspect involves directing the first output flow through a second flow path in a second zone of the flow reactor having a temperature $T^2$, to provide a second output flow comprising TNT.

The second zone of the flow reactor is suitably a reaction zone, as described above. Said reaction zone may be referred to as a second reaction zone. The second zone suitably comprises at least one residence plate. The second zone may comprise at least one further residence plate, such that the residence plate and the further residence plate are maintained at the temperature $T^2$. The number and sizes of said residence plates may be adjusted according to the desired volume and/or residence time of the first output flow in the second flow path.

In the second flow path of the second zone of the flow reactor, at least a second reaction suitably takes place. In said second reaction in the second flow path of the second zone of the flow reactor, the DNT comprised in the first output flow is suitable nitrated to produce the TNT in the second output flow. The second output flow suitably comprises at least 90 mol% TNT, at least 93 mol% or at least 95 mol% TNT, with respect to all toluene-derived species present in the second output flow.

The temperature $T^2$ is suitably a temperature sufficient to promote and the reaction of DNT in the first output flow with the nitric acid and sulphuric acid to produce TNT. The temperature $T^2$ may be at least 100°C, at least 110°C or at least 120°C. The temperature $T^2$ may be up to 230°C, up to 200°C, up to 180°C up to 160°C or up to 150°C.

The temperature $T^2$ is suitably from 100 to 230°C or from 110 to 170°C. The temperature $T^2$ may be from 120 to 160°C or from 125 to 155°C or from 125 to 150°C. For example, $T^2$ may be approximately 130°C.

In the method of this first aspect, the temperature $T^2$ is greater than the temperature $T^1$. Suitably, the temperature $T^2$ is at least 10°C greater than the temperature $T^1$. The temperature $T^2$ may be at least 20°C greater than the temperature $T^1$. The temperature $T^2$ may be at least 30°C, at least 40°C or at least 50°C greater than the temperature $T^1$.

In some embodiments, $T^1$ is from 20 to 100°C and $T^2$ is from 110 to 170°C.

In some embodiments, $T^1$ is from 60 to 90°C and $T^2$ is from 120 to 160°C.

In some embodiments, $T^1$ is from 75 to 85°C and $T^2$ is from 125 to 155°C.

It will be appreciated that the method of the first aspect can be carried out on flow reactors with differing configurations, particularly in relation to flow path lengths, volumes of said flow paths, flow rates through said flow paths and therefore residence times in said flow paths.

In the flow reactor used to carry out the method, the first flow path has a volume $V^1$ and the second flow path has a volume $V^2$. $V^1$ and $V^2$ may be varied according to the scale of TNT production required of the method. However, the inventors have found that it may be advantageous to the performance of the method for $V^2$ to be larger than $V^1$. Suitably the volume $V^2$ is at least 1.5 times larger than the volume $V^1$. The volume $V^2$ may be at least twice the volume $V^1$. The volume $V^2$ may be at least three times the volume $V^1$.

In the flow reactor used to carry out the method, the first flow path has a residence time $R^1$ and the second flow path has a residence time $R^2$. Suitably the residence time $R^2$ is larger than the residence time $R^1$. Suitably the residence time $R^2$ is at least 1.5 times larger than the residence time $R^1$. The residence time $R^2$ may be at least twice the residence time $R^1$. The residence time $R^2$ may be at least three times the residence time $R^1$.

The method of this first aspect may involve applying a positive backpressure to the flow reactor. A suitable backpressure may be applied using a peristaltic pump or a backpressure regulator (BPR), for example, which may be positioned on the output line. The amount of backpressure applied may depend on the scale of the flow reactor and the scale at which the method is carried out. The amount of backpressure applied is suitably sufficient to suppress the formation of gas pockets which otherwise form in the first flow path and/or the second flow path of the flow reactor, during operation of the method, which may otherwise adversely affect the yield of TNT produced by the method. For example, the backpressure applied my be at least 2 barg, at least 3 barg, at least 4 barg or at least 5 barg.

The method of this first aspect may involve a step:

f) collecting the second output flow.

The second output flow may be collected to provide the product TNT using any suitable means known in the art.

Step f) of collecting the second output flow may involve directing the second output flow into a vessel comprising water to precipitate the TNT. The precipitated TNT may then be collected by filtration and optionally washed, for example with water. The TNT obtained in this manner may have a purity suitable for direct further use, for example a purity of at least 90%, at least 93% or at least 95%.

The method of this first aspect may provide a yield of TNT of at least 50% with respect to the toluene starting material, suitably at least 60% or at least 65%. In some embodiments, the method may provide a yield of TNT of at least 70%.

The yield and purity of TNT provided by the method of this first aspect may be advantageously higher than the yield and purity achieved by known methods.

In some embodiments, there is provided a method for the synthesis of 2,4,6-trinitrotoluene (TNT) using a flow reactor, the method comprising the steps of:

    a) providing an input flow of sulphuric acid comprising at least 90 wt% sulphuric acid;

    b) providing an input flow of nitric acid comprising at least 90 wt% nitric acid;

    c) providing an input flow of toluene;

    d1) combining the input flow of sulphuric acid and the input flow of nitric acid to provide a combined acid flow;

    d2) combining the combined acid flow with the input flow of toluene to provide a first reaction flow;

    d3) directing the first reaction flow through a first flow path in a first zone of the flow reactor, the first zone having a temperature $T^1$ of from 75 to 85°C, to provide a first output flow;

e) directing the first output flow through a second flow path in a second zone of the flow reactor, the second zone having a temperature $T^2$ of from 125 to 155°C to provide a second output flow comprising TNT;

wherein the input flow of nitric acid provides from 5 to 7 equivalents of nitric acid, compared to the toluene provided by the input flow of toluene.

According to a second aspect of the present invention, there is provided a method for the synthesis of an energetic material comprising at least three nitro groups, the method using a flow reactor, the method comprising the steps of:

a) providing an input flow of sulphuric acid comprising at least 50 wt% sulphuric acid;

b) providing an input flow of nitric acid comprising at least 50 wt% nitric acid;

c) providing an input flow of an energetic material precursor;

d1) combining the input flow of sulphuric acid and the input flow of nitric acid to provide a combined acid flow;

d2) combining the combined acid flow with the input flow of toluene to provide a first reaction flow;

d3) directing the first reaction flow through a first flow path in a first zone of the flow reactor, the first zone having a temperature $T^1$, to provide a first output flow;

e) directing the first output flow through a second flow path in a second zone of the flow reactor, the second zone having a temperature $T^2$, to provide a second output flow comprising the energetic material comprising nitro groups;

wherein the temperature $T^2$ is greater than the temperature $T^1$.

The of this second aspect of the present invention may have any of the suitable features and advantages described above in relation to the first aspect.

The energetic material precursor provided in step c) may be selected according to the desired energetic material to be produced by the method. The person skilled in the relevant art would be able to select such appropriate energetic material precursors.

BRIEF DESCRIPTION OF THE FIGURES

Embodiments of the invention will now be described by way of example only with reference to the figures, in which:

Figure 1 shows a schematic of a flow reactor arranged in configuration 1 discussed below, to carry out a method of the first aspect of the present invention.

Figure 2 shows a schematic of a flow reactor arranged in configuration 2 discussed below, to carry out a method of the first aspect of the present invention.

EXAMPLES

Experimental reagents

Toluene (≥99%) was purchased from Fisher Scientific.
Sulphuric acid (95-97%) was obtained from Sigma-Aldrich.
Nitric acid (69%) was purchased from VWR Chemicals and concentrated nitric acid (≥99%) from Honeywell.
1H NMR spectroscopy was performed on the Magritek Spinsolve 60 and Bruker AVA500 (60 MHz and 500 MHz, respectively) at 298 K. The experiments were conducted using chloroform-D (obtained from Sigma-Aldrich) and referenced to the residual solvent peak ($\delta$ 7.26 ppm).
Thermograms were recorded in the temperature range 40 to 100°C at 10 K min-1, using a TA Instruments DSC 2500 Discovery Series calorimeter and crimped Tzero aluminium pans with a pierced lid. The sample masses were approximately 1 mg.

Flow Reactions

Reactions were carried out in a Protrix flow reactor with three Vapourtec SF10 peristaltic pumps fitted to the long side of the reactor (inlet port A; blue pump tube, inlet ports B1/B2; red pump tube). The mixer and residence modules were maintained at different temperatures, $T^1$ and $T^2$ respectively, by separating the modules with a spacer plate. Water was used as the thermal fluid for the mixer module and silicone oil as the thermal fluid for the residence modules.

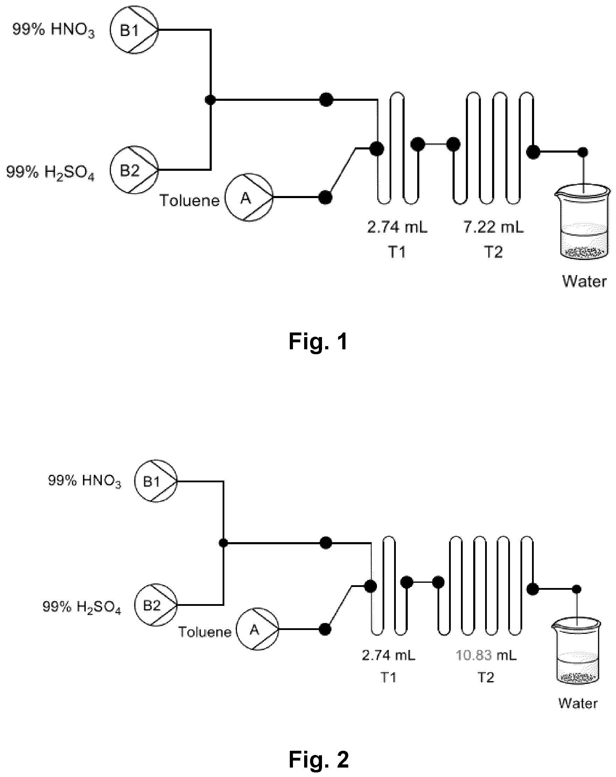

Configuration 1 (depicted in Figure 1) - Long side of the reactor only and two residence modules used.

Configuration 2 (depicted in Figure 2) - Long side of the reactor only and three residence modules used.

The reactions were carried out as follows with reference to Figures 1 and 2. Nitric acid (99%) was pumped (at a flow rate of 0.236 ml/min) into port B1 of the mixer (MRXP) module (i.e. the first zone of the flow reactor) and sulphuric acid (98%) (at a flow rate of 1.660 mL/min) into port B2 to generate the mixed acid nitrating agent (i.e. the combined acid flow). The reaction was initiated by introducing toluene (at a flow rate of 0.100 mL/min) into the flowing acid mixture via port A, to provide a first reaction flow. The Protrix reactor was configured with a spacer plate between the mixer module (first zone) and two residence (MRHP) modules arranged to provide a reaction zone (i.e. the second zone of the flow reactor). The spacer plate between the first and second zones allows said zones to be maintained at the different temperatures, $T^1$ and $T^2$, respectively. The first reaction flow was pumped through the long sections of the mixer and residence modules (first and second zones).

In configuration 1 (Figure 1), the flow reactor comprised a 2.74 mL first zone at $T^1$ ($T^1 = 80°C$) and a 7.22 mL second zone at $T^2$ (see Table 1).

In configuration 2 (Figure 2), the flow reactor comprised a 2.74 mL first zone at $T^1$ ($T^1 = 80°C$) and a 10.83 mL second zone at $T^2$ (see Table 1).

The product port (P) was connected to a Vapourtec SF10 pump operating as a BPR, and the product mixture was collected in a conical flask containing deionised water (DI) water. The collection vessel was cooled in an ice-water bath. Following collection, the product suspension was stirred to ensure complete precipitation of the product. The solid was collected by gravity filtration onto a preweighed filter paper and washed thoroughly with DI. The solid and filter paper were left to dry overnight then weighed to determine the product yield. The product was analysed by $^1$H NMR spectroscopy to determine the relative abundance of DNT and TNT, and thus estimate the purity of the material.

The assignments of the $^1$H NMR signals are provided below:

2,4,6-trinitrotoluene (TNT): 1H NMR (500 MHz, CDCl3): $\delta$ 8.84 (s, 2H, CH), 2.71 (s, 3H, CH3).

2,4-dinitrotoluene (2,4-DNT): 1H NMR (500 MHz, CDCl3): $\delta$ 8.83 (d, 1H, CH), 8.36 (dd, 1H, CH), 7.54 (dd, 1H, CH), 2.73 (s, 3H, CH3).

2,6-dinitrotoluene (2,6-DNT): 1H NMR (500 MHz, CDCl3): $\delta$ 8.00 (d, 2H, CH), 7.51 (t, 1H, CH), 2.58 (s, 3H, CH3).

To estimate the purity of the product, the integrals of the signals at 8.80-8.90 ppm ($I_1$), 8.30-8.40 ppm ($I_2$) and 7.55-7.65 ppm ($I_3$) were compared. Signal $I_1$ relates to a combination of TNT (2H) and 2,4-DNT (1H), $I_2$ relates to 2,4-DNT (1H), therefore $I_1$-$I_2$ represents an integration of 2 H in TNT. $I_3$ is believed to represent both DNT isomers corresponding to 1 H each. The relative molar quantity of DNT, $x_{DNT}$, is equated to $I_2$, as the other values are affected by overlapping signals. Since $I_1$ comprises contributions from one DNT aryl proton and the two equivalent aryl protons of TNT, the relative molar quantity of TNT, $x_{TNT}$, is equated to half the difference between $I_1$ and $x_{DNT}$. Thus:

$$\text{Purity (mol\%)} = \frac{x_{TNT}}{x_{TNT} + x_{DNT}} \times 100\%$$

$$= \frac{(I_1 - I_2)/2}{(I_1 - I_2)/2 + I_2} \times 100\% = \frac{I_1 - I_2}{I_1 + I_2} \times 100\%$$

The experiments shown in Table 1 below were carried out as described above, with the variations in the flow reactor configuration, temperature $T^2$ and backpressure noted in the Table. Where noted in the Table, a backpressure was applied to the reactor via a peristaltic pump, or backpressure regulator (BPR), on the outlet line. The yields and purity of the TNT produced by these reactions are noted. As noted above, $T^1 = 80°C$ in each experiment.

Table 1

| Ex. | Config. | $T^1$ (°C) | $T^2$ (°C) | Backpressure (barg) | Product mass (g) | Purity (%)t | TNT yield (%) |
|---|---|---|---|---|---|---|---|
| 1 | 1 | 80 | 80 | - | 0.7284 | 1.5 | 1.0 |
| 2 | 1 | 80 | 100 | - | 0.8789 | 7.0 | 5.7 |
| 3 | 1 | 80 | 120 | - | 0.7875 | 48.3 | 35.6 |
| 4 | 1 | 80 | 80 | 3.1 | 0.8068 | 1.5 | 1.1 |
| 5 | 1 | 80 | 100 | 3.1 | 0.7820 | 26.2 | 19.2 |
| 6 | 1 | 80 | 120 | 3.1 | 0.7180 | 62.0 | 41.6 |
| 7 | 1 | 80 | 80 | 5.7 | 0.8196 | 2.0 | 1.5 |
| 8 | 1 | 80 | 100 | 5.7 | 0.7956 | 24.0 | 17.8 |
| 9 | 1 | 80 | 120 | 5.7 | 0.8207 | 61.7 | 47.4 |
| 10 | 1 | 80 | 130 | 5.7 | 0.7598 | 80.3 | 57.1 |
| 11 | 1 | 80 | 140 | 5.7 | 0.7238 | 95.6 | 64.8 |
| 12 | 1 | 80 | 150 | 5.7 | 0.5724 | 98.1 | 52.6 |
| 13 | 2 | 80 | 130 | 5.7 | 0.7185 | 95.2 | 64.0 |
| 14 | 2 | 80 | 140 | 5.7 | 0.7723 | 96.2 | 69.6 |
| 15 | 2 | 80 | 150 | 5.7 | 0.7002 | 99.1 | 64.9 |
| 16[a] | 2 | 80 | 130 | 5.7 | 0.7881 | 95.2 | 70.2 |
| 17[a] | 2 | 80 | 140 | 5.7 | 0.8197 | 97.9 | 75.1 |
| 18[a] | 2 | 80 | 150 | 5.7 | 0.8022 | 98.1 | 73.7 |
| 19[b] | 2 | 80 | 130 | 5.7 | 4.1364 | 95.5 | 61.6 |
| 20[ab] | 2 | 80 | 130 | 5.7 | 4.0327 | 95.9 | 60.3 |

The error in the backpressure was $\pm$ 0.2 bar for all experiments.

†TNT purity determined by 1H NMR spectroscopy based only on the DNT and TNT integrals.

[a] Freshly distilled nitric acid used.

[b] 30 minute collection time.

To assess the effect of varying the temperature $T^2$ on the TNT yield and purity, the reaction was first performed at a single temperature by setting $T^1$ = $T^2$ = 80 °C (Example 1). Based on the integrals of DNT and TNT only, TNT represented just 2% of the product. When $T^2$ was raised to 120°C, this value increased to 48% (Examples 2 and 3). However, increasing $T^2$ also results in significant gas formation, lowering the overall residence time. By applying a backpressure in the reactor via a peristaltic pump, or backpressure regulator (BPR), on the outlet line, the gas pockets in the line were suppressed and the residence time maintained at the target value of ~5 minutes. The increased residence time resulted in an increase in TNT purity, from 48% with no applied backpressure (Examples 1-3) to 62% with 3.1 barg pressure (Examples 4-6). Raising the pressure again to 5.7 barg (Examples 7-9) did not produce a further increase in TNT purity.

In the reactions involving configuration 2, TNT purity increased with $T^2$. The yield increased to a maximum at $T^2$ = 140°C, as in configuration 1, and fell slightly when the temperature was increased further. The target 95% molar purity can be achieved at $T^2$ = 130°C.

Nitric acid can gradually decompose on storage, even under ambient conditions, to produce nitrogen dioxide, oxygen and water. Dissolved nitrogen dioxide dimerises reversibly to form dinitrogen tetroxide, causing the otherwise colourless nitric acid to become yellow or orange in colour. The concentration of the acid is reduced by this process, which can suppress the formation of the nitronium ($NO_2^+$) ion necessary for the desired nitration reaction. The effect of the age of the nitric acid used in the above reaction was therefore investigated by repeating experiments 13-15 using freshly prepared nitric acid (Examples 16-18). The use of the fresh nitric acid resulted in an increase in TNT yield and did not significantly affect the purity of the TNT product.

Further experiments with the flow reactor in configuration 2 were conducted to test the effects of varying the relative quantities of the acids.

Firstly, Example 13 was repeated with smaller quantities of sulphuric acid (Table 2). The flow rates of the other reactants were increased (as noted) to maintain a constant nitric acid / toluene ratio (6 eq.) and a total flow rate of 2.00 mL min-1. The other reaction parameters were also left unchanged, with the aim of achieving the target TNT purity of 95%.

The following parameters were used for said experiments (Examples 21-24):

$T^1 = 80°C$

$T^2 = 130°C$

Backpressure = 5.7 ± 0.2 barg

Collection time = 5 minutes

The results are shown in Table 2 below.

Table 2

| Ex. | Config. | sulphuric acid / toluene molar ratio (eq.) | toluene | nitric acid | sulphuric acid | Purity (%)† | TNT yield (%) |
|-----|---------|---------------------------------------------|---------|-------------|----------------|-------------|---------------|
| 13 | 2 | 33.0 | 0.100 | 0.236 | 1.660 | 95.2 | 64.0 |
| 21 | 2 | 30.0 | 0.108 | 0.255 | 1.637 | 88.5 | 63.7 |
| 22 | 2 | 27.5 | 0.116 | 0.274 | 1.610 | 88.1 | 65.1 |
| 23 | 2 | 25.0 | 0.125 | 0.295 | 1.580 | 92.6 | 67.7 |
| 24 | 2 | 22.5 | 0.136 | 0.320 | 1.540 | 92.1 | 65.4 |

†TNT purity determined by 1H NMR spectroscopy based only on the DNT and TNT integrals.

Lowering the relative amount of sulphuric acid used in the reaction mixture did not significantly affect the yield of TNT. Therefore it may be possible to reduce the relative amounts of sulphuric acid used in the method, which may improve the economic viability of the TNT production method. However, the purity of the TNT was somewhat reduced in some cases.

Secondly, an experiment was conducted to test the effect of varying the nitric acid quantity on the outcome of the reaction (Example 25). The flow rates of the other reactants were increased to maintain a constant sulphuric acid / toluene ratio (33 eq.) and a total flow rate of 2.00 mL min-1.

The following parameters were used for said experiments:

$T^1 = 80°C$

$T^2 = 130°C$

Backpressure = 5.7 ± 0.2 barg

Collection time = 5 minutes

The results are shown in Table 3 below.

Table 3

| Ex. | Config. | nitric acid / toluene molar ratio (eq.) | toluene | nitirc acid | sulphuric acid | Purity (%)† | TNT yield (%) |
|-----|---------|------------------------------------------|---------|-------------|----------------|-------------|---------------|
| 13 | 2 | 6.0 | 0.100 | 0.236 | 1.6600 | 95.2 | 64.0 |
| 25 | 2 | 4.5 | 0.103 | 0.182 | 1.7150 | 67.8 | 48.0 |

†TNT purity determined by 1H NMR spectroscopy based only on the DNT and TNT integrals.

The reaction (Example 25) wherein the equivalents of nitric acid used were reduced to 4.5 afforded a significantly reduced yield and purity of TNT. NMR analysis of the product determined ~32% DNT was present in the product, which suggests that the reaction did not reach completion.

The results shown in Tables 1, 2 and 3 demonstrate that TNT can be successfully synthesised from toluene in a continuous flow process to obtain relatively high yield and purity of TNT, using the method of the present invention. In particular, the use of a process involving the formation of DNT from toluene in a first reaction zone at a temperature $T^1$ and the formation of TNT from DNT in a second reaction zone at a temperature $T^2$, wherein the temperature $T^2$ is higher than the temperature $T^1$, can provide TNT which does not contain significant amounts of unreacted DNT. This method of the present invention may therefore provide the potential safety, logistic and cost advantages discussed herein, when implemented on a commercial scale.

**Claims**

1. A method for the synthesis of 2,4,6-trinitrotoluene (TNT) using a flow reactor, the method comprising the steps of:

   a) providing an input flow of sulphuric acid comprising at least 50 wt% sulphuric acid;
   b) providing an input flow of nitric acid comprising at least 50 wt% nitric acid;
   c) providing an input flow of toluene;
   d) directing the input flow of sulphuric acid, the input flow of nitric acid and the input flow of toluene through a first flow path in a first zone of the flow reactor, the first zone having a temperature $T^1$, to provide a first output flow;
   e) directing the first output flow through a second flow path in a second zone of the flow reactor, the second zone having a temperature $T^2$, to provide a second output flow comprising TNT;

   wherein the temperature $T^2$ is greater than the temperature $T^1$.

2. The method according to claim 1, wherein the input flow of sulphuric acid comprises at least 90% sulphuric acid.

3. The method according to claim 1 or claim 2, wherein the input flow of nitric acid comprises at least 90% nitric acid.

4. The method according to any preceding claim, wherein the input flow of nitric acid and the input flow of toluene provide at least 5 equivalents of nitric acid compared to toluene.

5. The method according to any preceding claim, wherein the first zone of the flow reactor is a mixing zone comprising a mixing plate and wherein the step d) involves mixing the input flow of sulphuric acid, the input flow of nitric acid and the input flow of toluene.

6. The method according to any preceding claim, wherein the temperature $T^1$ is from 30 to 90°C.

7. The method according to any preceding claim, wherein step d) involves combining the input flow of sulphuric acid and the input flow of nitric acid to provide a combined acid flow, and involves combining the combined acid flow with the input flow of toluene.

8. The method according to any preceding claim, wherein the first output flow comprises dinitrotoluene (DNT).

9. The method according to any preceding claim, wherein the temperature $T^2$ is from 120 to 160°C.

10. The method according to any preceding claim, wherein the temperature $T^2$ is at least 20°C greater than the temperature $T^1$.

11. The method according to any preceding claim, wherein the first flow path has a volume $V^1$ and the second flow path has a volume $V^2$, wherein the volume $V^2$ is at least twice the volume $V^1$.

12. The method according to any preceding claim, wherein the first flow path has a residence time $R^1$ and the second flow path has a residence time $R^2$, wherein the residence time $R^2$ is at least twice the residence time $R^1$.

13. The method according to any preceding claim, wherein the method involves a step:
    f) collecting the second output flow.

14. The method according to claim 13, where step f) involves directing the second output flow into a vessel comprising water to precipitate the TNT.

**15.** A method for the synthesis of an energetic material comprising at least three nitro groups, the method using a flow reactor, the method comprising the steps of:

a) providing an input flow of sulphuric acid comprising at least 50 wt% sulphuric acid;
b) providing an input flow of nitric acid comprising at least 50 wt% nitric acid;
c) providing an input flow of an energetic material precursor;
d) directing the input flow of sulphuric acid, the input flow of nitric acid and the input flow of energetic material precursor through a first flow path in a first zone of the flow reactor, the first zone having a temperature $T^1$, to provide a first output flow;
e) directing the first output flow through a second flow path in a second zone of the flow reactor, the second zone having a temperature $T^2$, to provide a second output flow comprising the energetic material comprising nitro groups;

wherein the temperature $T^2$ is greater than the temperature $T^1$.

99% HNO₃ (B1)

99% H₂SO₄ (B2)

Toluene (A)

2.74 mL
T1

7.22 mL
T2

Water

**Fig. 1**

99% HNO₃ (B1)

99% H₂SO₄ (B2)

Toluene (A)

2.74 mL
T1

10.83 mL
T2

Water

**Fig. 2**

Europäisches
Patentamt

European
Patent Office

Office européen
des brevets

# EUROPEAN SEARCH REPORT

Application Number

EP 25 27 5007

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 3 053 908 A (KOUBA DELORE L ET AL) 11 September 1962 (1962-09-11) * claim 1 * * examples 1-3 * * examples 4-7 * ----- | 1-15 | INV. C07C201/08 C07C205/06 |
| A | KYPRIANOU DIMITRIS ET AL: "Synthesis of 2,4,6-Trinitrotoluene (TNT) Using Flow Chemistry", MOLECULES, vol. 25, no. 16, 6 August 2020 (2020-08-06), page 3586, XP093049756, DE ISSN: 1433-1373, DOI: 10.3390/molecules25163586 * abstract * * tables 1, 2 * ----- | 1-15 | |
| A | CN 101 544 567 B (DALIAN CHEMICAL PHYSICS INST) 25 July 2012 (2012-07-25) * example 1 * ----- | 1-15 | |
| A | US 4 022 844 A (DE CAZENOVE HUBERT E ET AL) 10 May 1977 (1977-05-10) * column 2, line 33 - line 45 * * column 5, table * ----- | 1-15 | |

TECHNICAL FIELDS SEARCHED (IPC)

C07C

The present search report has been drawn up for all claims

| Place of search | Date of completion of the search | Examiner |
|---|---|---|
| The Hague | 12 July 2025 | Karolak, Ewelina |

## ANNEX TO THE EUROPEAN SEARCH REPORT
## ON EUROPEAN PATENT APPLICATION NO.

EP 25 27 5007

This annex lists the patent family members relating to the patent documents cited in the above-mentioned European search report.
The members are as contained in the European Patent Office EDP file on
The European Patent Office is in no way liable for these particulars which are merely given for the purpose of information.

12-07-2025

| Patent document cited in search report | | Publication date | Patent family member(s) | | Publication date |
|---|---|---|---|---|---|
| US 3053908 | A | 11-09-1962 | NONE | | |
| CN 101544567 | B | 25-07-2012 | NONE | | |
| US 4022844 | A | 10-05-1977 | DE | 2309544 A1 | 31-10-1973 |
| | | | ES | 411958 A1 | 01-06-1976 |
| | | | FR | 2173454 A5 | 05-10-1973 |
| | | | GB | 1368544 A | 25-09-1974 |
| | | | US | 4022844 A | 10-05-1977 |

EPO FORM P0459

For more details about this annex : see Official Journal of the European Patent Office, No. 12/82